Europäisches Patentamt

European Patent Office

Office européen des brevets

(19)

(11) Veröffentlichungsnummer : **0 141 223**
**B1**

(12) # EUROPÄISCHE PATENTSCHRIFT

(45) Veröffentlichungstag der Patentschrift :
21.10.87

(21) Anmeldenummer : 84111208.9

(22) Anmeldetag : 20.09.84

(51) Int. Cl.⁴ : **C 12 N 11/08, C 12 N 11/02, C 12 N 11/06, C 12 N 9/48, C 12 P 41/00**

(54) Verfahren zur Herstellung einer immobilisierten Acylase und ihre Verwendung.

(30) Priorität : 27.09.83 DE 3334846

(43) Veröffentlichungstag der Anmeldung :
15.05.85 Patentblatt 85/20

(45) Bekanntmachung des Hinweises auf die Patenterteilung : 21.10.87 Patentblatt 87/43

(84) Benannte Vertragsstaaten :
AT BE CH DE FR GB IT LI NL SE

(56) Entgegenhaltungen :
EP-A- 0 037 667
EP-A- 0 052 365
DE-A- 3 131 908
DE-B- 2 336 829

(73) Patentinhaber : HOECHST AKTIENGESELLSCHAFT
Postfach 80 03 20
D-6230 Frankfurt am Main 80 (DE)

(72) Erfinder : Grabley, Susanne, Dr.
Hölderlinstrasse 7
D-6240 Königstein/Taunus (DE)
Erfinder : Keller, Reinhold, Dr.
Wiesenweg 5
D-6232 Bad Soden am Taunus (DE)
Erfinder : Schlingmann, Merten, Dr.
Schneidhainer Strasse 32a
D-6240 Königstein/Taunus (DE)

**Beschreibung**

Die Immobilisierung von Acylasen ist schon mehrfach beschrieben worden : in der Britischen Patentschrift 1 369 462 ist beschrieben, daß man N-Acyl-D,L-aminosäuren mit Acylasen umsetzen kann, wobei selektiv die L-Aminosäure umgesetzt wird. Es wird erwähnt, daß die Acylase auch auf einem inerten Material wie Cellulose, Sand oder Aluminiumoxid adsorbiert sein kann und mit einem bifunktionalen Reagenz wie Glutardialdehyd vernetzt werden kann. In der Deutschen Offenlegungsschrift 2 732 301 wird die Immobilisierung von Proteinen beschrieben, wobei speziell auf das Enzym Penicillinacylase verwiesen wird. Nach der Deutschen Patentschrift 3 048 612 kann eine derart fixierte Penicillin-G-Acylase zur Racematspaltung von D,L-2-Amino-4-methylphosphinobuttersäure eingesetzt werden.

Die Europäische Patentanmeldung A1-43169 betrifft Enzympräparationen mit Inulinase-Aktivität. In einer bevorzugten Ausführungsform wird das Enzym durch Reaktion mit Glutardialdehyd an ein makroporöses Harz mit Amino- und/oder Hydroxygruppen gebunden. Als Beispiel wird ein Phenol-Formaldehyd-Harz mit Hydroxygruppen aufgeführt und erwähnt, daß auch ein Phenol-Formaldehyd-Harz mit Aminogruppen Verwendung finden könne. Weiterhin wird erwähnt, daß zur Bindung des Enzyms an einen Träger als bifunktionale Reagenzien neben Dialdehyden auch Dicarbonsäuren, deren Anhydride oder Diisocyanate in Betracht kommen.

In der EP-A1-43169 ist der Phenol-Formaldehyd-Harz-Träger mit Aminogruppen durch das Warenzeichen [R]DUOLITE A-7 charakterisiert. Nach der Europäischen Patentanmeldung A2-37667 handelt es sich hierbei um ein makroporöses Anion-Austauscherharz auf Phenol-Formaldehydbasis mit einem Teilchendurchmesser von 250-840 $\mu$m, 31 m$^2$/g spezifischer Oberfläche, 0,52 cm$^3$/g Gesamtvolumen der Makroporen, die einen Porendurchmesser von 10-200 nm aufweisen, und 7,5 meq/g Anion-Austauscherkapazität aufgrund von primären und sekundären Aminogruppen. Dieses Produkt wird von der Firma Diamond Shamrock vertrieben. Als allgemein geeignet werden granulat- oder perlförmige Anion-Austauscherharze auf Phenol-Formaldehydbasis beschrieben, deren Größe vorzugsweise im Bereich von 250 $\mu$m bis 1,4 mm liegt.

In der Deutschen Offenlegungsschrift 3 131 908 wird ein Verfahren beschrieben, nach dem Aminoacylase an ein poröses Phenolharz, z. B. ein Phenol/Formaldehyd-Kondensat mittels eines Dialdehyds immobilisiert wird.

Es wurde nun gefunden, daß sich Anion-Austauscherharze auf Phenol-Formaldehyd-Basis mit im wesentlichen sekundären Aminogruppen besonders gut zur Fixierung von Acylasen eignen, wobei das Enzym mit Hilfe eines Diisocyanats an den Träger gebunden wird. Von besonderem Vorteil ist, daß zur Bindung des Enzyms an den Träger kein Katalysator erforderlich ist, wie es beispielsweise bei den in der Europäischen Patentanmeldung EP-A1-52365 genannten Methoden der Fall ist.

Als Diisocyanate kommen aromatische, cycloaliphatische und insbesondere aliphatische Diisocyanate in Betracht, wie sie beispielsweise in der EP-A1-52365 beschrieben sind. Bevorzugt sind handelsübliche aliphatische Diisocyanate wie das 2,2,4- und 2,4,4-Trimethyl-hexamethylen-1,6-diisocyanat und insbesondere das Hexamethylen-1,6-diisocyanat.

Als Acylasen kommen die bekannten zur Penicillinspaltung und Racematspaltung von N-Acylaminosäuren verwendeten Produkte in Betracht, wie sie in der Britischen Patentschrift 1 369 462, den Deutschen Offenlegungsschriften 2 352 579, 2 446 320 oder 2 939 269 und insbesondere in der Deutschen Patentschrift 3 048 612 beschrieben sind.

Zur Kopplung des Enzyms an den Ionenaustauscher wird dieser zunächst in die freie Basenform überführt, zweckmäßig durch Suspendieren in einer starken wäßrigen Base wie Natronlauge. Nach Neutralwaschen und Trocknen wird das wasserfreie Harz in einem aprotischen Lösemittel für das Diisocyanat aufgenommen und das Diisocyanat in Substanz oder in Lösung zugesetzt und gut vermischt. Das Umsetzungsprodukt kann nach Abtrennen von der Reaktionslösung und Waschen mit dem Lösemittel unmittelbar zu der Umsetzung mit dem Enzym verwendet werden. Es kann aber auch isoliert und getrocknet werden und ist unter Feuchtigkeitsausschluß längere Zeit haltbar.

Das so vorbehandelte Harz kann unmittelbar mit einer wäßrigen Lösung des Enzyms umgesetzt werden, wobei sich die Reaktionsbedingungen nach den Eigenschaften des Enzyms richten. Im allgemeinen wird das vorbehandelte Harz mit der wäßrigen Enzymlösung ausreichend lange bei Raumtemperatur verrührt, das Produkt abfiltriert und gewaschen, beispielsweise mit gesättigter oder physiologischer Kochsalzlösung und anschließend mit Wasser. Das gewaschene Enzymimmobilisat kann unmittelbar eingesetzt werden.

Eine erfindungsgemäß immobilisierte Penicillin-G-Acylase aus E. coli zeigt gegenüber einem nach der Deutschen Offenlegungsschrift 2 732 301 erhaltenen Immobilisat eine stark erhöhte Aktivität, was sich beispielsweise darin zeigt, daß auch ein sterisch gehindertes Substrat wie N-Acyl-D,L-tert.-Leucin quantitativ und stereospezifisch gespalten wird.

Die Erfindung betrifft deshalb auch die Verwendung einer erfindungsgemäß immobilisierten Penicillin-G-Acylase zur stereospezifischen Spaltung von N-Acyl-D,L-aminosäuren.

In den folgenden Beispielen werden bevorzugte Ausgestaltungen der Erfindung näher erläutert. Prozentangaben beziehen sich auf das Gewicht, wenn nichts anderes angegeben ist.

Beispiel 1

Das handelsübliche makroporöse Anionen-Austauscherharz auf Basis Phenol-Formaldehyd mit im wesentlichen sekundären Aminogruppen DUOLITE A-7 wird zur Überführung in die freie Basenform in 2 N Natronlauge aufgenommen, gerührt und mit entionisiertem Wasser neutral gewaschen. Nach Trocknen im Vakuumschrank (40 °C, 50 mbar, 24 Stunden) werden 100 g des wasserfreien Harzes in 200 ml wasserfreiem Dichlormethan aufgenommen, mit 10 g Hexamethylen-1,6-diisocyanat versetzt und 2 Stunden unter Feuchtigkeitsausschluß bei Raumtemperatur gerührt. Anschließend filtriert man ab und wäscht das Harz mit Dichlormethan gründlich nach. Das Harz wird unter Feuchtigkeitsausschluß von Dichlormethan befreit und unter Feuchtigkeitsausschluß bis zum Einsatz aufbewahrt.

Beispiel 2

Das nach Beispiel 1 erhaltene Harz wird in 200 ml einer wäßrigen Lösung von 10 g Penicillin-G-Acylase (E.C. Nr. 3.5.1.11, aus E. coli ATCC 11 105) gegeben und 2 Stunden bei Raumtemperatur gerührt. Das Produkt wird abfiltriert und zuerst mit gesättigter Kochsalzlösung und anschließend mit entionisiertem Wasser gewaschen.

Beispiel 3

Die spezifische Aktivität des nach Beispiel 2 erhaltenen Penicillin-G-Amidase-Immobilisats wird gegenüber dem Standard-Substrat Penicillin-G wie folgt bestimmt :
1 g Penicillin-G-Kaliumsalz wird in 40 ml Wasser gelöst und unter Rühren bei 37 °C mit 100 mg Immobilisat versetzt. Durch Konstanthalten des pH-Werts auf 7,80 kann durch Titration mit 0,1 N Natronlauge aus dem Alkaliverbrauch direkt die umgesetzte Menge an Penicillin-G zu 6-Aminopenicillansäure und Phenylessigsäure abgelesen werden.
Nach 30 Minuten Reaktionszeit sind 3,6 ml 0,1 N Natronlauge entsprechend 360 μMol Penicillin-G verbraucht. Hieraus errechnet sich die spezifische Aktivität des Immobilisats zu 120 U/g.

Beispiel 4

10 g (33,4 mMol) N-Phenacetyl-D,L-2-amino-4-methylphosphinobuttersäure werden in 40 ml Wasser gelöst. Die Lösung, die einen pH-Wert von 7,8 aufweist, wird bei 37 °C mit einem Gramm Penicillin-G-Amidase-Immobilisat gemäß Beispiel 2 unter Rühren versetzt und der pH-Wert durch Zugabe von 0,1 N Natronlauge konstant auf 7,8 gehalten. Nach 5 bis 6 Stunden ist die Reaktion beendet. Nach üblicher Aufarbeitung (Deutsche Patentschrift 3 048 612) erhält man 3,5 g (16 mMol, entsprechend 96 % der Theorie) L-2-Amino-4-methylphosphinobuttersäure vom Schmelzpunkt 199-201 °C, $[\alpha]^{20}$ 24° (c = 1, in 1 N Salzsäure).

Beispiel 5

100 g (0,4 Mol) N-Phenacetyl-D,L-tert.-Leucin werden in 400 ml 1 N Natronlauge gelöst und auf einen pH-Wert von 7,8 und ein Gesamtvolumen von 1 000 ml eingestellt. Diese Reaktionslösung wird auf 37 °C thermostatisiert und unter Rühren mit 100 g nach Beispiel 2 immobilisierter Penicillin-G-Amidase versetzt. Durch Titrieren mit 0,05 N Natronlauge wird der pH-Wert während der Hydrolysereaktion konstant gehalten und gleichzeitig der Umsetzungsverlauf registriert. Nach 3 bis 4 Stunden wird keine Natronlauge mehr verbraucht. Nach Abfiltrieren des Enzymmaterials und Ansäuern mit konzentrierter Schwefelsäure auf pH 3 kristallisieren Phenylessigsäure und N-Phenacetyl-D-tert.-Leucin aus. Die Phenylessigsäure kann durch Extraktion mit Essigsäureethylester-Hexan aus dem Kristallisat abgetrennt werden, worauf 45 g (0,18 Mol = 90 % d. Th.) N-Phenacetyl-D-tert.-Leucin vom Schmelzpunkt 163 bis 164 °C, $[\alpha]_D^{20}$ 14,2° (c = 1, in Methanol) zurückbleiben.
Aus der eingeengten Mutterlauge werden durch Extraktion mit Ethanol-Wasser 23 g (0,18 Mol = 90 % d. Th.) L-tert.-Leucin vom Schmelzpunkt über 250 °C, $[\alpha]_D^{20}$ — 9,4° (c = 1, in Wasser) isoliert.

**Patentansprüche**

1. Verfahren zur Herstellung einer immobilisierten Acylase, dadurch gekennzeichnet, daß man das Enzym mittels eines Diisocyanats an ein makroporöses Anionaustauscherharz auf Basis Phenol-Formaldehyd mit im wesentlichen sekundären Aminogruppen bindet.

2. Verfahren nach Anspruch 1, dadurch gekennzeichnet, daß das Trägerharz einen Porendurchmesser von 10 bis 200 Nanometer und einen Teilchendurchmesser von 250 bis 840 μm aufweist.

3. Verfahren nach Anspruch 1 oder 2, dadurch gekennzeichnet, daß das Diisocyanat ein Alkylendiisocyanat mit bis zu 10 Kohlenstoffatomen in der Alkylenkette ist.

4. Verfahren nach einem oder mehreren der vorhergehenden Ansprüche, dadurch gekennzeichnet, daß das Enzym Penicillin-G-Acylase ist.

5. Verfahren nach Anspruch 1 bis 4, dadurch gekennzeichnet, daß das Trägerharz in der freien Basenform mit dem Diisocyanat umgesetzt wird und das Reaktionsprodukt in wäßrigem Medium mit einer Lösung des Enzyms umgesetzt wird.

6. Verwendung der nach Anspruch 4 erhältlichen Acylase zur stereospezifischen Spaltung von N-Acyl-D,L-aminosäuren.

**Claims**

1. A process for the preparation of an immobilized acylase, wherein the enzyme is bound by means of a diisocyanate to a macroporous anion exchange resin based on phenol/formaldehyde and carrying essentially secondary amino groups.

2. The process as claimed in claim 1, wherein the carrier resin has a pore diameter of 10 to 200 nanometers and a particle diameter of 250 to 840 µm.

3. The process as claimed in claim 1 or 2, wherein the diisocyanate is an alkylene diisocyanate having up to 10 carbon atoms in the alkylene chain.

4. The process as claimed in one or more of the preceding claims, wherein the enzyme is penicillin-G acylase.

5. The process as claimed in claims 1 to 4, wherein the carrier resin in the free base form is reacted with the diisocyanate and the reaction product is reacted in an aqueous medium with a solution of the enzyme.

6. The use of the acylase obtainable as claimed in claim 4 for the cleavage of amide bonds.

**Revendications**

1. Procédé pour la préparation d'une acylase immobilisée, caractérisé en ce que l'on fixe l'enzyme, au moyen d'un diisocyanate, sur une résine macroporeuse échangeuse d'anions, à base de phénol et formaldéhyde, comportant des groupes amino essentiellement secondaires.

2. Procédé selon la revendication 1, caractérisé en ce que la résine de support présente un diamètre de pores de 10 à 200 nm et un diamètre de particules de 250 à 840 µm.

3. Procédé selon la revendication 1 ou 2, caractérisé en ce que le diisocyanate est un alkylènediisocyanate ayant jusqu'à 10 atomes de carbone dans la chaîne alkylène.

4. Procédé selon une ou plusieurs des revendications précédentes, caractérisé en ce que l'enzyme est la pénicilline G acylase.

5. Procédé selon les revendications 1 à 4, caractérisé en ce que l'on fait réagir la résine de support, sous la forme de base libre, avec le diisocyanate, et on fait réagir le produit de réaction, en milieu aqueux, avec une solution de l'enzyme.

6. Utilisation de l'acylase préparable selon la revendication 4, pour le dédoublement stéréospécifique de N-acyl-D,L-amino-acides.